# EUROPÄISCHE PATENTSCHRIFT

(11) **EP 0 477 902 B1**
(45) Veröffentlichungstag und Bekanntmachung des Hinweises auf die Patenterteilung: **11.02.1998**
(21) Anmeldenummer: 91116335.0
(22) Anmeldetag: 25.09.1991
(51) Int. Cl.: C12P 13/04, C12P 13/14, C12P 7/46

(54) **Verfahren zur Herstellung von L-Phosphinothricin durch eine gekoppelte enzymatische Reaktion**
Process for the preparation of L-Phosphinothricine by a coupled enzymatic reaction
Procédé de préparation de L-Phosphinothricine par une réaction enzymatique combinée

(30) Priorität: 27.09.1990 DE 4030578
(43) Veröffentlichungstag der Anmeldung: 01.04.1992
(73) Patentinhaber: Hoechst Schering AgrEvo GmbH, 13342 Berlin (DE)
(72) Erfinder: Bartsch, Klaus, Dr., W-6374 Steinbach (DE); Fülling, Gerd, Dr., W-6230 Frankfurt am Main (DE); Schulz, Arno, Dr., W-6239 Eppstein/Taunus (DE)

(56) Entgegenhaltungen:
- EP-A- 0 135 846
- EP-A- 0 249 188
- WO-A-87/01727

## Beschreibung

Die Erfindung betrifft die Synthese von L-2-Amino-4-(hydroxymethylphosphinyl)-buttersäure (L-Phosphinothricin) aus 4-(Hydroxymethylphosphinyl)-2-oxo-buttersäure (HMPB) durch Kopplung zweier enzymatischer Reaktionen.

Die Herstellung von Phosphinothricin in einer gekoppelten enzymatischen Synthese ist bereits aus der EP 249 188 bekannt. Darin wird die Umsetzung von α-Ketoglutarat in Glutamat in Anwesenheit des Aminodonors Aspartat und einer Glutamat/Oxalacetat-Transaminase (GOT) beschrieben. Diese Umsetzung ist mit einer weiteren Transaminasereaktion gekoppelt, die 4-(Hydroxymethylphosphinyl)-2-oxo-buttersäure (HMPB) zu L-Phosphinothricin in Anwesenheit des in der ersten Reaktion gebildeten Aminodonors Glutamat umsetzt.

Für einen vollständigen Substratumsatz bei gekoppelten Enzymreaktionen ist es nun wichtig, daß die Gleichgewichtskonstanten der einzelnen Enzymreaktionen voneinander verschieden sind. Bei Transaminierungsreaktionen ist jedoch die Gleichgewichtskonstante in etwa 1,0, so daß das gewünschte Produkt im allgemeinen nur in einer 50-%igen Ausbeute entstehen kann (U.S.-Pat. 4,826,766).

Um das Gleichgewicht von enzymatischen Reaktionen zugunsten der Produkte zu verschieben, kann man entweder ein Reaktionsprodukt aus dem Gleichgewicht entfernen, oder eine Ausgangssubstanz in molarem Überschuß einsetzen.

Für die genannte Transaminierungsreaktion zur Synthese von L-Phosphinothricin setzt man im allgemeinen den Aminodonor Glutamat in hohem Überschuß ein [A. Schulz et al. (1990) Appl. Environ. Micobiol. 56, 1-6, Nr. 1]. Dies hat jedoch den Nachteil, daß die nicht-proteinogene Aminosäure L-Phosphinothricin von den natürlichen Aminosäuren, wie z.B. Glutamat, nur sehr aufwendig, beispielsweise durch zwei nachfolgende Ionenaustauscher-Chromatographien [A. Schulz et al. (1990) Appl. Environ. Micobiol. 56, 1-6 Nr. 1], abgetrennt werden kann.

Alternativ kann man in einem gekoppelten Prozess, an dem eine phosphinothricinspezifische Transaminase und eine GOT beteiligt sind, durch "Recycling" des von der Phosphinothricin-Transaminase verbrauchten Glutamats durch die GOT die Reaktion in Richtung Phosphinothricin-Synthese treiben, da das Reaktionsprodukt der GOT, Oxalacetat, spontan in Anwesenheit zweiwertiger Metallionen zu Pyruvat decarboxyliert und somit aus dem Reaktionsgleichgewicht entfernt wird. Allerdings haben alle bisher beschriebenen GOT's eine Nebenaktivität, die Pyruvat in Anwesenheit von Glutamat in Alanin transaminiert, so daß der Reaktion kontinuierlich NH₄⁺ entzogen wird und die Reaktion bei Einsatz äquimolarer Mengen HMPB und Aminodonor (Glutamat und/oder Aspartat) nicht vollständig zum L-Phosphinothricin ablaufen kann. Zusätzlich wird das entstehende L-Phosphinothricin durch Alanin verunreinigt.

Es wurde nun gefunden, daß in einer gekoppelten Enzymreaktion mit einer Transaminase mit GOT-Aktivität und mit einer Transaminase mit L-Phosphinothricin-Transaminaseaktivität Phosphinothricin in nahezu quantitativer Ausbeute und praktisch ohne jegliche Verunreinigung durch eine natürliche Aminosäure entsteht, wenn der Aminodonor Glutamat in katalytischen Mengen und der Aminodonor Aspartat in etwa äquimolaren Mengen bezogen auf HMPB eingesetzt wird und die Transaminasen Pyruvat nicht zu Alanin umsetzen.

Gegenstand der Erfindung ist daher:

Ein Verfahren zur Herstellung von L-2-Amimo-4-(hydroxymethylphosphinyl)-buttersäure (L-Phosphinothricin) der Formel (I) 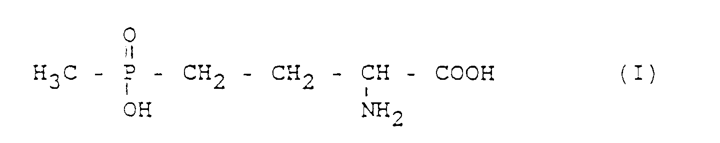 aus 4-(Hydroxymethylphosphinyl)-2-oxo-buttersäure (HMPB) der Formel (II) 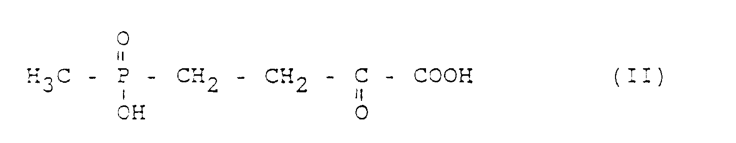 in einer gekoppelten Enzymreaktion bestehend aus folgenden Schritten:
a) Umsetzung von Aspartat und α-Ketoglutarat in Anwesenheit einer geeigneten Transaminase 1 zu Oxalacetat und Glutamat und
b) Umsetzung von Glutamat und HMPB der Formel (II) in Anwesenheit einer geeigneten Transaminase 2 zu α-Ketoglutamat und L-Phosphinothricin, das
dadurch gekennzeichnet ist, daß das molare Verhältnis von Aspartat zu HMPB 0,5-1,5 zu 1, vorzugsweise 0,8-1,2 zu 1, insbesondere äquimolar ist und Glutamat oder α-Ketoglutarat in katalytischen Mengen zugegeben wird und die Transaminasen Pyruvate nicht zu Alanin umsetzen.

Glutamat, Aspartat und α-Ketoglutarat bzw. ihre korrespondierenden Säuren sind käuflich erhältliche Substanzen. HMPB läßt sich nach EP 30424 herstellen.

Die Transaminase 1 ist jedes Enzym mit Transaminaseaktivität, das α-Ketoglutarat in Gegenwart von Aspartat als Aminodonor in Glutamat transminieren kann (sogenanntes Enzym mit GOT-Aktivität). Als Transaminase 1 wird eine GOT (Glutamat/Oxalacetat-Transaminase) verwendet, die Pyruvat nicht zu Alanin transaminieren kann und auf diese Weise das gewünschte Produkt der gekoppelten Reaktion, L-Phosphinothricin, durch eine weitere natürliche Aminosäure nicht verunreinigen würde. Pyruvat entsteht beispielsweise durch Decarboxylierung von Oxalacetat, dem Transaminierungsprodukt von Aspartat.

Insbesondere können Transaminasen mit GOT-Aktivität, aus Escherichia coli (E. coli) oder aus Bakterien der Gattung Bacillus verwendet werden, die keine pyruvat-spezifische Aktivität haben.

Die Transaminase 2 ist jedes Enzym mit Transaminaseaktivität, das HMPB zu L-Phosphinothricin in Gegenwart von Glutamat als Aminodonor transaminieren kann. Derartige Enzyme sind in EP 249 188 angegeben. Als Transaminase 2, analog wie bei der Transaminase 1, wird eine Transaminase verwendet, die Pyruvat nicht zu Alanin transaminieren kann.

Insbesondere die L-Phosphinothricin-spezifische Transaminase aus E. coli hat keine pyruvat-spezifische Aktivität und kann daher bevorzugt verwendet werden. Die L-Phosphinothricin-spezifische Transaminase aus E. coli kann beispielsweise nach A. Schulz (1990) angereichert werden.

Grunsätzlich können für die Transaminierungsreaktionen auch ganze Zellen mit der jeweiligen Transaminaseaktivität, Zellextrakte, teilweise gereinigte Zellextrakte oder gereinigte Enzyme verwendet werden. Es ist jedoch vorteilhaft, die Enzyme soweit gereinigt einzusetzen, daß keine Nebenreaktionen er auftreten, insbesondere die Transaminierung von Pyruvat zu Alanin.

3esonders vorteilhaft ist es, mindestens ein Enzym, insbesondere beide, in immobilisierter Form einzusetzen. Ein mögliches Verfahren zur Immobilisierung von Transaminasen wird beispielsweise von A. Schulz et al. (1990) beschrieben.

Die Transaminierungsreaktionen werden im allgemeinen in einem biokompatiblen Puffer durchgeführt, d.h. In einem Puffer, der den pH-Wert in einem Bereich von 6,5 bis 10, vorzugsweise 7,5 bis 9,0, insbesondere 7,5 bis 8,5, aufrechterhält und mit den einzelnen Reaktionspartnern nicht reagiert. Vorzugsweise wird ein Phosphat- oder Tris-Puffer, insbesondere ein Tris-Puffer, gewählt. Das molare Verhältnis von Aspartat zu HMPB ist 0,5-1,5 zu 1, vorzugsweise 0,8-1,2 zu 1, insbesondere äquimolar. Glutamat oder α-Ketoglutarat wird in katalytischen Mengen den Reaktionsansatz zugegeben. Im allgemeinen ist das Verhältnis von Glutamat oder α-Ketoglutarat zu HMPB 0,01-1 zu 1, vorzugsweise 0,01-0,2 zu 1, insbesondere 0,05-0,2 zu 1. Die Reaktionsansätze enthalten im allgemeinen noch geringe Mengen des Cofaktors Pyridoxalphosphat, beispielsweise in einer Konzentration von 1-500 µM, vorzugsweise 5-100 µM. Die Reaktionstemperatur liegt im allgemeinen zwischen ca. 20 und 70 °C, vorzugsweise von 30 bis 40 °C.

Zur Steigerung der Ausbeute an L-Phosphinothricin wird vorzugsweise das aus der Glutamat/Oxalacetat-Transaminasereaktion entstandene Oxalacetat in Anwesenheit von mehrwertigen Metallionen decarboxyliert (UK Patent Application 2 161 159). Als mehrwertige Metallionen kommen beispielsweise alle in der britischen Patentanmeldung aufgeführten Metallionen in Frage, vorzugsweise Al³⁺, Mg²⁺, Mn²⁺, Fe²⁺ oder Fe³⁺. Bei dieser bevorzugten Ausführungsform ist es weiterhin von Vorteil, keine Transaminase oder Transaminasefraktion mit einer zusätzlichen pyruvat-spezifischen Aktivität zu verwenden. Die Steigerung der Ausbeute an L-Phosphinothricin durch die Decarboxylierungsreaktion von Oxalacetat bei den angegebenen Reaktionsbedingungen war völlig unerwartet, da nach der UK Patent Application 2 161 159 die höchste Produktausbeute nur mit Aspartat als einzigem Aminodonor in dem Reaktionssystem gefunden wurde.

Durch den praktisch vollständigen Verbrauch der Aminodonatoren Aspartat und Glutamat fällt das Produkt L-Phosphinothricin praktisch frei von natürlichen Aminosäuren an und kann von der gebildeten α-Ketosäuren α-Ketoglutarat und Oxalacetat bzw. Pyruvat in einem einzigen Schritt abgetrennt werden. Obwohl α-Ketoglutarat bzw. Glutamat nicht im Überschuß, sondern nur in sehr geringen Mengen und ebenso Aspartat nicht im Überschuß, sondern vorzugsweise äquimolar bezogen auf HMPB zugesetzt wurden, wurde dennoch eine praktisch vollständige Umsetzung von HMPB zu L-Phosphinothricin beobachtet. Dies war in keiner Weise vorherzusehen.

Die Reinigung von L-Phosphinothricin kann nach bekannten Verfahren beispielsweise durch Extraktion mit Methylisobutylketon oder über eine Kationenaustauscherchromatographie, wie z.B. mit Amberlite™ IR 120, einfach durchgeführt werden.

Das gewonnene L-Phosphinothricin wird im allgemeinen in der Landwirtschaft als Herbizid eingesetzt. Die anschließenden Beispiele sollen die Erfindung in ihren verschiedenen Aspekten näher erläutern, ohne sie in irgendeiner Weise einzuschränken.

### Beispiel 1:

### Synthese von L-Phosphinothricin durch Biotransformation mit Escherichia coli K-12

E. coli K-12 wurde in LB-Medium (10 g/l Trypton, 5 g/l Hefe-Extrakt, 10 g/l NaCl) für 15 h bei 37 °C kultiviert. Die Bakterienzellen wurden durch Zentrifugation für 10 min. bei 5000 g geerntet, zweimal in Phosphatpuffer (10 mM NaPhosphat, pH = 7,0, 10 mM NaCl) gewaschen und anschließend mit einer Konzentration von 100 mg/ml in folgenden Lösungen resuspendiert:

### Lösung 1:

| HMPB: Glutaminsäure : Asparaginsäure = 1 : 1 : 1 | |
|---|---|
| 100 mM | HMPB |
| 100 mM | Glutaminsäure |
| 100 mM | Asparaginsäure |
| 50 mM | Tris/HCl, pH = 8,0. |

### Lösung 2:

| HMPB: Glutaminsäure = 1:4 | |
|---|---|
| 100 mM | HMPB |
| 400 mM | Glutaminsäure |
| 50 mM | Tris/HCl, pH = 8,0. |

Die Zellsuspensionen wurden mit den Reaktionsgemischen bei 37 °C inkubiert. Der Gehalt an L-Phosphinothricin in den Reaktionsansätzen wurde zu verschiedenen Zeiten während des Syntheseverlaufs mit dem Aminosäureanalysator bestimmt. In der Tabelle 1 ist der %-Umsatz an L-Phosphinothricin während des Reaktionsverlaufs für beide Biotransformationsansätze dargestellt. Während bei vierfachem Überschuß des Aminodonors Glutaminsäure (Lösung 2) die L-PPT-Ausbeute 85 % nicht überschritt, konnten bei äquimolarem Einsatz der Aminodonatoren Glutaminsäure und Asparaginsäure (Lösung 1) Umsatzraten von nahezu 100 % erzielt werden.

**Tabelle 1:**

| L-Phosphinothricin-Produktion mit Zellen von E. coli K-12 | | |
|---|---|---|
| L-Phosphinothricin (%-Umsatz) Reaktionsgemische | | |
| Zeit (h) | HMPB:Glu:Asp=1 : 1 : 1 | HMPB :Glu=1:4 |
| 2 | 1 | 2 |
| 4 | 3 | 4 |
| 24 | 23 | 36 |
| 48 | 71 | 72 |
| 120 | 94 | 78 |
| 165 | 96 | 82 |
| 218 | 98 | 86 |

Die folgende Beispiele sollen den Einsatz von mehrwertigen Kationen und die Immobilisierung von Transaminasen erläutern.

### Beispiel 2:

Reinigung von Glutamat/Oxalacetat Transaminase aus Schwein

### Beispiel 3:

### Synthese von L-Phosphinothricin mit gereinigter L-Phosphinothricin-spezifischer Transaminase aus E. coli und GOT aus Schwein bei unterschiedlichen Glutaminsäurekonzentrationen

Die L-Phosphinothricin-spezifische Transaminase (PST) aus E. coli K-12 (siehe EP-A 0 344 683) und die GOT aus Schweineherzen (siehe Beispiel 2) wurden in Phosphatpuffer (2o mM NaPhosphat, pH = 7,0, 0,1 mM Pyridoxalphosphat, 1 mM 2-Ketoglutarat, 5 mM β-Mercaptoethanol) mit einer Konzentration von 1 mg/ml gelöst. Anschließend wurden jeweils die einer Aktivität von 1 unit (= 1 µmol L-Phosphinothricin/min bei PST bzw 1 µmol Glutamat/min. bei GOT) entsprechenden Volumina der beiden Enzyme gemischt und mit folgenden Reaktionslösungen bei 37 °C für 1 h inkubiert:

Alle Reaktionsgemische enthielten

| | |
|---|---|
| 100 mM | HMPB |
| 100 mM | Asparaginsäure |
| 50 mM | Tris/HCl, pH = 8,0 |

und zusätzlich

| | | |
|---|---|---|
| Glutaminsäure | in Versuch 1 | 100 mM |
| | in Versuch 2 | 50 mM |
| | in Versuch 3 | 20 mM |
| | in Versuch 4 | 10 mM |
| | in Versuch 5 | 5 mM |
| | in Versuch 6 | - |
| | | |

Der Gehalt an L-Phosphinothricin in den verschiedenen Ansätzen nach Ablauf der Reaktionszeit wurde mit dem Aminosäureanalysator (Biotronic™ LC 5001) bestimmt. Die daraus als %-Umsatz ermittelten L-Phosphinothricin-Syntheseraten sind in Tabelle 2 zusammengestellt. Die Experimente zeigen, daß die Glutaminsäurekonzentration bis auf das 0,2fache des eingesetzten HMPB bzw. Aspartat erniedrigt werden kann, ohne daß die Geschwindigkeit der L-Phosphinothricin-Synthese nennenswert beeinträchtigt wird.

**Tabelle 2:**

| L-Phosphinothricin-Synthese bei verschiedenen Glutamatkonzentrationen | | |
|---|---|---|
| Versuch | Reaktionsgemisch | L-PPT (%-Umsatz/h) |
| 1 | HMPB:Glu:Asp=1:1:1 | 55,5 |
| 2 | HMPB:Glu:Asp=1:0,5:1 | 48,5 |
| 3 | HMPB:Glu:Asp=1:0,2:1 | 43,5 |
| 4 | HMPB:Glu:Asp=1:0,1:1 | 30,6 |
| 5 | HMPB:Glu:Asp=1:0,05:1 | 18,9 |
| 6 | HMPB:Asp=1:1 | 3,0 |

### Beispiel 4:

### Synthese von L-Phosphinothricin mit gereinigter PST (E. coli) und GOT (Schwein) bei unterschiedlichen Enzymverhältnissen

Die beiden gereinigten Transaminasen wurden wie in Beispiel 3 beschrieben mit einer Reaktionslösung bestehend aus 100 mM HMPB, 20 mM Glutaminsäure, 100 mM Asparaginsäure und 50 mM Tris/HCl, pH = 8,0 bei 37 °C inkubiert. Dabei wurden verschiedene Aktivitätsverhältnisse der beiden Enzyme eingesetzt:

| | |
|---|---|
| 1. | 1 unit PST/10 units GOT |
| 2. | 1 unit PST/ 1 unit GOT |
| 3. | 1 unit PST/ 0 unit GOT |

Der Gehalt an L-Phosphinothricin in den Ansätzen wurde zu verschiedenen Zeitpunkten während des Ablaufs der Synthesereaktion durch Messung von Testproben im Aminosäureanalysator bestimmt. Die daraus ermittelten L-Phosphinothricin-Synthesen sind in der Tabelle 3 dargestellt. Der höchste Umsatz wurde mit dem Enzymverhältnis PST:GOT=1:1 erzielt (Ansatz 2). Ohne Zugabe der GOT (Ansatz 3) betrug die maximal erreichbare PPT-Umsatzrate nur ca. 20 %, da in diesem Fall nur die Glutaminsäure, nicht aber die Asparaginsäure für die Synthese von L-Phosphinothricin genutzt werden kann.

**Tabelle 3:**

| L-PPT-Synthese mit verschiedenen Enzymverhältnissen | | | |
|---|---|---|---|
| Zeit (h) | PST:GOT=1:10 (Ansatz 1) | %-Umsatz PST:GOT=1:1 (Ansatz 2) | PST:GOT:1:0 (Ansatz 3) |
| 1 | 29 | 31 | 16 |
| 2 | 35 | 39 | 18 |
| 4 | 44 | 50 | 18 |
| 8 | 49 | 65 | 19 |
| 24 | 67 | 78 | 20 |
| 55 | 74 | 86 | 20 |

### Beispiel 5:

Einfluß von Manganchlorid auf die L-Phosphinothricin-Synthese

Die gereinigten Transaminasen wurden wie im Beispiel 3 beschrieben im Verhältnis 1:1 (je 1 unit) gemischt und mit Reaktionslösung (100 mM HMPB, 20 mM Glutaminsäure, 100 mM Asparaginsäure, 50 mM Tris/HCl, pH = 8,0) in Anwesenheit von 1 mM MnCl₂ bzw. ohne MnCl₂ bei 37 °C inkubiert. Der Ablauf der L-Phosphinothricin-Synthese wurde wie in Beispiel 4 beschrieben gemessen und ist in der Tabelle 4 zusammengefaßt. Durch Zugabe von Manganchlorid konnte die Reaktionsgeschwindigkeit deutlich gesteigert werden. Nach 24 h Reaktionszeit lag der L-Phosphinothricin-Umsatz um etwa 10 % über dem des Vergleichsansatzes ohne Manganchlorid.

**Tabelle 4:**

| Einfluß von Manganchlorid auf die L-PPT-Synthesereaktion | | |
|---|---|---|
| Zeit (h) | %-Umsatz | |
| | + 1 mM MnCl₂ | - MnCl₂ |
| 1 | 30 | 26 |
| 2 | 41 | 35 |
| 4 | 53 | 45 |
| 8 | 64 | 58 |
| 24 | 88 | 76 |

### Beispiel 6:

### Immobilisierung der Transaminase "PST" an Kieselgel

Zu 50 ml PST-Lösung (gereinigte PST aus E. coli, 10 mg Protein/ml Lösung in 0,25 M Kaliumphosphatpuffer, pH 8) gibt man 50 ml aktiviertes Kieselgel (Silanierung und Glutardialdehyd-Aktivierung nach Literatur: K. Mosbach, Methods in Enzymology, Vol. XLIV; Academic Press, New York, 1976, S. 139 u. 140) und läßt unter leichtem Rühren für 3 h reagieren. Anschließend filtriert man den noch feuchten Katalysator und wäscht mit 100 ml 0,25 M Phosphatpuffer, pH 8. Der so hergestellte Biokatalysator kann für die unten beschriebenen Reaktionen eingesetzt werden.

### Beispiel 7

### Immobilisierung der Transaminase "GOT" an Kieselgel

10 ml einer GOT-Lösung (gereinigte GOT aus dem Schwein, 8,4 mg Protein/10 ml Lösung) werden wie in Beispiel 6 beschrieben an 10 ml Kieselgel immobilisiert. Der Biokatalysator kann in dieser Form für die gekoppelte Transaminierung eingesetzt werden.

### Beispiel 8

### Gekoppelte Transaminierung mit immobilisierter PST und immobilisierter GOT

180 mg (1 mmol) HMPB, 133 mg (1 mmol) L-Asparaginsäure, 29,4 mg (0,2 mmol) L-Glutaminsäure, 10 mg Pyridoxalphosphat und 0,6 g TRIS werden mit dest. Wasser auf 5 g Lösung aufgefüllt und auf pH 8 gestellt. Dazu gibt man 0,5 ml immobilisierte PST (Beispiel 6) sowie 0,5 ml immobilisierte GOT (Beispiel 7) und läßt unter vorsichtigem Rühren bei pH 8 für 48 h bei 26°C reagieren. Die Reaktionsmischung wird per HPLC (Aminosäureanalysator) auf Aminosäuregehalt untersucht.

| | |
|---|---|
| Ausbeute (HPLC): | 0,122 g L-PPT (68 % bezogen auf HMPB) |
| | 0,012 g L-Asparaginsäure |
| | 0,028 g L-Glutaminsäure |
| | 0,025 g L-Alanin |

### Beispiel 9

### Gekoppelte Transaminierung mit immobilisierter PST und immobilisierter GOT

1 g (5,6 mmol) HMPB, 715 mg (5,6 mmol) L-Asparaginsäure, 163 mg (1,1 mmol) L-Glutaminsäure und 10 mg Pyridoxalphosphat werden mit dest. Wasser auf 20 g Lösung aufgefüllt und mit KHCO₃ auf pH 8 gestellt. Nach Zugabe von 1 ml immobilisierter PST (Beispiel 6) und 1 ml GOT (Beispiel 7) läßt man unter leichtem Rühren bei 36°C für 72 h reagieren. Die Produktlösung wird per HPLC (ASA) auf Aminosäuregehalt untersucht.

| | |
|---|---|
| Ausbeute (HPLC): | 0,58 g L-PPT (58 % bezogen auf HMPB) |
| | 0,30 g L-Asparaginsäure |
| | 0,16 g L-Glutaminsäure |
| | 0,02 g L-Alanin |

### Beispiel 10

### Gekoppelte Transaminierung mit immobilisierter PST und freier GOT

180 mg (1 mmol) HMPB, 147 mg (1 mmol) L-Glutaminsäure, 133 mg (1 mmol) L-Asparaginsäure, 10 mg Pyridoxalphosphat und 0,7 g TRIS werden mit dest. Wasser auf 50 g Lösung, pH 8 aufgefüllt und 0,5 ml immobilisierte PST aus der Lösung gemäß Beispiel 6 sowie 42 µl freie GOT aus der gemäß Beispiel 7 hergestellten GOT Lösung zugefügt. Die Umsetzung erfolgt unter vorsichtigem Rühren bei 36°C. Nach 48 h wird der Aminosäuregehalt der Lösung per HPLC (ASA) überprüft.

| | |
|---|---|
| Ausbeute (HPLC): | 0,145 g L-PPT (80 % bezogen auf HMPB) |
| | 0,024 g L-Asparaginsäure |
| | 0,147 g L-Glutaminsäure |
| | 0,003 g L-Alanin |

### Beispiel 11

### Gekoppelte Transaminierung mit immobilisierter PST und immobilisierter GOT

Die Umsetzung wird analog Beispiel 10 durchgeführt. Es wird 0,5 ml immobilisierte GOT (Beispiel 6) anstelle der freien GOT eingesetzt. Die Zusammensetzung der Aminosäuren in der Produktlösung entspricht der von Beispiel 10.

Der L-PPT-Gehalt wurde dünnschichtchromatographisch ermittelt. Er liegt bei ca. 80 %.

## Patentansprüche

1. Verfahren zur Herstellung von L-2-Amino-4-(hydroxyphosphinyl)-buttersäure (L-Phosphinothricin) aus4-(Hydroxymethylphosphinyl)-2-oxobuttersäure (HMPB) in einer gekoppelten Enzymreaktion bestehend aus folgenden Schritten:
a) Umsetzung von Aspartat und alpha-Ketoglutarat in Anwesenheit einer geeigneten Transaminase 1 zu Oxalacetat und Glutamat und
b) Umsetzung von Glutamat und HMPB in Anwesenheit einer geeigneten Transaminase 2 zu alpha-Ketoglutarat und L-Phosphinothricin,
dadurch gekennzeichnet, daß das molare Verhältnis von Aspartat zu HMPB 0,5-1,5 zu 1, vorzugsweise 0,8-1,2 zu 1, insbesondere äquimolar ist, Glutamat oder alpha-Ketoglutarat in katalytischen Mengen zugegeben wird und die Transaminasen Pyruvat nicht zu Alanin umsetzen.

2. Verfahren nach Anspruch 1, dadurch gekennzeichnet, daß Glutamat oder α-Ketoglutarat in einem molaren Verhältnis von 0,01-1 zu 1, vorzugsweise 0,01-0,2 zu 1, insbesondere 0,05-0,2 zu 1 bezogen auf HMPB zugegeben wird.

3. Verfahren nach Anspruch 1 oder 2, dadurch gekennzeichnet, daß die Transaminase 1 aus Escherichia coli oder Bacillus stammt.

4. Verfahren nach Anspruch 1 oder 2, dadurch gekennzeichnet, daß die Transaminase 2 eine L-Phosphinothricin-Transaminaseaktivität besitzt.

5. Verfahren nach Anspruch 4, dadurch gekennzeichnet, daß die L-Phosphinothricin-spezifische Transaminase aus Escherichia coli stammt.

6. Verfahren nach mindestens einem der Ansprüche 1 bis 5, dadurch gekennzeichnet, daß Oxalacetat in Anwesenheit von mehrwertigen Kationen, vorzugsweise Al³⁺, Mg²⁺, Mn²⁺, Zn²⁺, Fe²⁺ oder Fe³⁺, zu Pyruvat decarboxyliert wird.

7. Verfahren nach mindestens einem der Ansprüche 1 bis 6, dadurch gekennzeichnet, daß mindestens eine Transaminase in immobilisierter Form vcrliegt.

8. Verfahren nach Anspruch 7, dadurch gekennzeichnet, daß die immobilisierten Transaminasen in einem Säulenreaktor vorliegen.

## Claims

1. A process for the preparation of L-2-amino-4-(hydroxymethylphosphinyl)butyric acid (L-phosphinothricin) from 4-(hydroxymethylphosphinyl)2-oxobutyric acid (HMPB) in a coupled enzyme reaction comprising the following steps:
a) reaction of aspartate and alpha-ketoglutarate in the presence of a suitable transaminase 1 to give oxalacetate and glutamate and
b) reaction of glutamate and HMPB in the presence of a suitable transaminase 2 to give alpha-ketoglutarate and L-phosphinothricin,
wherein the molar ratio of aspartate to HMPB is 0.5-1.5 to 1, preferably 0.8-1.2 to 1, in particular equimolar, glutamate or alpha-ketoglutarate is added in catalytic amounts, and the transaminases do not convert pyruvate into alanine.

2. The process as claimed in claim 1, wherein glutamate or α-ketoglutarate is added in a molar ratio of 0.01-1 to 1, preferably 0.01-0.2 to 1, in particular 0.05-0.2 to 1 relative to HMPB.

3. The process as claimed in claim 1 or 2, wherein the transaminase 1 originates from Escherichia coli or Bacillus.

4. The process as claimed in claim 1 or 2, wherein the transaminase 2 has an L-phosphinothricin transaminase activity.

5. The process as claimed in claim 4, wherein the L-phosphinothricin-specific transaminase originates from Escherichia coli.

6. The process as claimed in at least one of claims 1 to 5, wherein oxalacetate is decarboxylated to pyruvate in the presence of multiply charged cations, preferably Al³⁺, Mg²⁺, Mn²⁺, Zn²⁺, Fe²⁺ or Fe³⁺.

7. The process as claimed in at least one of claims 1 to 6, wherein at least one transaminase is present in immobilized form.

8. The process as claimed in claim 7, wherein the immobilized transaminases are present in a column reactor.

## Revendications

1. Procédé de préparation de l'acide L-2-amino-4-(hydroxyméthylphosphinyl)butyrique (L-phosphùlothricine) à partir de l'acide 4-(hydroxyméthylphosphb1yl)-2-oxobutyrique (HMPB) dans une réaction enzymatique couplée constituée des étapes suivantes:
a) la transformation d'aspartate et d'a--cétoglutarate en oxalacétate et en glutamate en présence d'une transaminase 1 appropriée et
b) la transformation de glutamate et de HMPB en α-cétoglutarate et L-phosphinothricine en présence d'une transaminase 2 appropriée,
caractérisé en ce que le rapport molaire de l'aspartate au HMPB est de 0,5 - 1,5 à 1, de préférence de 0,8 - 1,2 à 1, en particulier équimolaire, en ce que l'on ajoute le glutamate ou l'a-cétoglutarate en des quantités catalytiques, et en ce que les transaminases ne transforment pas le pyruvate en alanine.

2. Procédé selon la revendication 1, caractérisé en ce que l'on ajoute le glutamate ou l'a-cétoglutarate en un rapport molaire de 0,01 - 1 à 1, de préférence de 0,01 - 0,2 à 1, en particulier de 0,05 - 0,2 à 1 par rapport au HMPB.

3. Procédé selon la revendication 1 ou 2, caractérisé en ce que la transaminase 1 provient *d'Escherichia coli* ou de *Bacillus.*

4. Procédé selon la revendication 1 ou 2, caractérisé en ce que la transaminase 2 possède une activité de L-phosphinothricine-transaminase.

5. Procédé selon la revendication 4, caractérisé en ce que la transaminase spécifique de la L-phosphinothricine provient *d'Escherichia coli.*

6. Procédé selon l'une au moins des revendications 1 à 5, caractérisé en ce que l'oxalacétate est décarboxylé en pyruvate en présence de cations polyvalents, de préférence Al³⁺, Mg²⁺, Mn²⁺, Zn²⁺, Fe²⁺ ou Fe³⁺.

7. Procédé selon l'une au moins des revendications 1 à 6, caractérisé en ce qu'au moins une transaminase se trouve sous forme immobilisée.

8. Procédé selon la revendication 7, caractérisé en ce que les transaminases immobilisées se trouvent dans un réacteur en colonne.
